# FASCICULE DE BREVET EUROPEEN

(11) **EP 1 332 734 B1**
(45) Date de publication et mention de la délivrance du brevet: **08.11.2006**
(21) Numéro de dépôt: 03356016.0
(22) Date de dépôt: 03.02.2003
(51) Int. Cl.: A61F 2/30, A61F 2/40, A61F 2/46

(54) **Elément prothétique comprenant deux composants et procédé d'assemblage d'un tel élément prothétique**
Aus zwei Bestandteilen bestehendes Prothesenelement und Verfahren zum Zusammensetzen eines solchen Prothesenelements
Prosthetic element comprising two components and process for assembling such a prosthetic element

(30) Priorité: 04.02.2002 FR 0201284
(43) Date de publication de la demande: 06.08.2003
(73) Titulaire: TORNIER S.A., 38330 Saint-Ismier (FR)
(72) Inventeur: Tornier, Alain, 38330 Saint Ismier (FR)
(74) Mandataire: Myon, Gérard Jean-Pierre

(56) Documents cités:
- EP-A- 0 552 950
- EP-A- 0 634 154
- EP-A- 0 815 808
- EP-A- 0 884 032
- WO-A-01/47442
- FR-A- 2 605 514
- FR-A- 2 689 756
- FR-A- 2 704 747
- FR-A- 2 737 107
- US-A- 6 136 032

## Description

L'invention a trait à un élément prothétique comprenant deux composants principaux, à une utilisation d'un tel élément et à des procédés d'assemblage d'un tel élément.

Un élément prothétique comprend le plus souvent une partie destinée à constituer une surface articulaire et une partie d'ancrage dans un os du patient. Compte tenu de leurs fonctions mécaniques respectives, ces parties sont parfois réalisées dans des composants distincts, ces composants étant chacun optimisés en ce qui concerne le choix de leurs matériaux constitutifs et leur mode de fabrication. Il convient alors de pouvoir assembler ces composants de façon précise et aisée. Par exemple, on peut fixer dans l'os un composant d'ancrage et rapporter sur celui-ci un composant formant surface articulaire.

Dans certains dispositifs antérieurs, tels que connus par exemple de FR-A-2 605 514, il est connu d'utiliser une vis de blocage d'une partie d'une prothèse sur une autre partie pourvue d'un taraudage correspondant. Il est également connu de FR-A-2 689 756 d'utiliser une vis de blocage d'une tige solidaire d'une tête articulaire à l'intérieur d'un alésage ménagé dans une partie d'ancrage de prothèse. On peut également prévoir, comme dans FR-A-2 737 107, d'utiliser une vis prisonnière d'un logement ménagé sur l'une des parties et destiné à coopérer avec un taraudage ménagé dans l'autre partie.

Dans le document WO 01 47442 est décrit un élément prothétique selon le préambule de la revendication 1.

Les dispositifs connus nécessitent un travail précis de positionnement et d'alignement des composants constitutifs d'un élément prothétique, ce qui n'est pas toujours possible car le champs opératoire est d'accès difficile et car il est délicat à visualiser du fait de l'enfoncement de la glène. En outre, les vis utilisées dans les systèmes connus peuvent être déplacées ou mal alignées par rapport aux parties avec lesquelles elles doivent coopérer, d'où un risque de fausser leur filet ou le taraudage avec lequel elles doivent coopérer.

C'est à ces inconvénients qu'entend plus particulièrement remédier l'invention en proposant un élément prothétique pouvant être assemblé de façon particulièrement aisée et précise, tout en demeurant très pratique à utiliser.

Dans cet esprit, l'invention concerne un élément prothétique qui comprend deux composants principaux destinés à être assemblés ensemble et immobilisés l'un par rapport à l'autre au moyen d'une vis, cet élément prothétique étant caractérisé en ce que la vis est apte à être vissée au moyen d'au moins un filet dans deux taraudages solidaires ou destinés à être rendus solidaires respectivement de l'un et de l'autre composants principaux. La vis comporte au moins un relief radial, apte à venir en appui contre une surface de butée solidaire d'un premier composant, et une extrémité non filetée formant doigt de guidage et apte à coulisser dans un logement ménagé dans le second composant.

Grâce à l'invention, la vis de blocage peut être immobilisée par rapport à l'un des composants principaux, ce qui garantit son positionnement et son orientation par rapport à celui-ci. Ensuite, lorsque son doigt de guidage est inséré dans le logement du second composant qui a une section transversale correspondante à celle du doigt, les composants sont guidés l'un par rapport à l'autre lors d'un mouvement d'approche mutuelle. Le relief radial prévu sur la vis lui permet d'exercer sur la surface de butée un effort dirigé vers le second composant, pour plaquer fermement ces composants l'un contre l'autre et contribuer ainsi à leur immobilisation relative, après vissage de la vis dans le second taraudage.

Selon des aspects avantageux mais non obligatoires de l'invention, cet élément prothétique incorpore une ou plusieurs des caractéristiques suivantes :
- le relief de la vis est formée par une collerette radiale ménagée sur la vis entre les deux filets de celle-ci.
- la surface de butée est annulaire et entoure cette vis. Dans ce cas, on peut prévoir que cette surface de butée est formée par une face d'un anneau entourant la vis, cet anneau étant rigidement lié à une douille vissée dans le premier composant. Cet anneau et cette douille permettent une transmission d'effort efficace entre la vis et le premier composant.
- les premier et second composants sont respectivement pourvus de surfaces de portée tronconiques, la vis étant apte à plaquer, par son vissage, ces surfaces l'une contre l'autre. Cet aspect de l'invention tire parti du fait que la vis de l'invention garantit un positionnement et un mouvement d'approche satisfaisants des surfaces de portée tronconiques prévues respectivement sur les premier et second composants, ce qui limite les risques de défaut d'alignement de ces surfaces. Ces surfaces de portée sont avantageusement respectivement centrées sur l'axe central de la vis, lorsque celle-ci est vissée dans le premier composant, et sur l'axe central du logement de coulissement du doigt de centrage.
- le taraudage solidaire ou destiné à être rendu solidaire du second composant est formé dans le logement de coulissement du doigt de centrage, du côté d'une zone d'entrée de la vis.
- le premier composant forme une surface articulaire convexe et définit un volume dans lequel peut être montée la vis et engagé, au moins partiellement, le second composant. Le premier composant peut, par exemple, être en forme de tronçon de sphère.
- le logement de coulissement du doigt de guidage est ménagé dans une tige d'ancrage du second composant dans un os.

Selon un premier mode de réalisation avantageux de l'invention, la vis est pourvue de deux filets aptes à coopérer successivement avec deux taraudages respectivement solidaires de l'un et de l'autre composants principaux.

Selon un second mode de réalisation avantageux de l'invention, la vis est pourvue d'un unique filet apte à coopérer successivement avec deux taraudages solidaires ou destinés à être rendus solidaires respectivement de l'un et de l'autre des composants principaux.

Une utilisation particulière d'un élément prothétique tel que précédemment décrit concerne la réalisation de la partie glénoïdienne d'une prothèse totale d'épaule.

L'invention concerne également un premier procédé d'assemblage d'un élément prothétique tel que précédemment décrit et, plus précisément, un procédé qui comprend des étapes consistant à :
- immobiliser la vis par rapport à un premier composant principal de l'élément prothétique par vissage de cette vis dans un premier taraudage solidaire ou destiné à être rendu solidaire du premier composant principal, une surface formant butée à un éloignement de la vis par rapport au premier composant étant disposé autour de cette vis postérieurement ou antérieurement à cette immobilisation ;
- coiffer avec un logement ménagé sur un second composant, une extrémité libre de la vis faisant saillie par rapport au premier composant ;
- pousser les premier et second composants l'un vers l'autre en faisant coulisser l'extrémité de la vis dans le logement précité ;
- dévisser la vis par rapport au premier taraudage et
- visser la vis dans un second taraudage ménagé dans le second composant, en exerçant sur la surface de butée précitée un effort de rapprochement des premier et second composants.

Selon un second procédé d'assemblage conforme à l'invention, on immobilise la vis et on coiffe son extrémité libre comme indiqué ci-dessus à la suite de quoi :
- on impacte l'un des composants principaux en direction de l'autre, de façon à les rapprocher en faisant coulisser l'extrémité libre de la vis dans le logement, jusqu'à mettre en contact les composants l'un avec l'autre ;
- on dévisse la vis par rapport au premier taraudage et
- on visse la vis dans un second taraudage ménagé dans le second composant jusqu'à obtenir un effort d'immobilisation relative des composants dans leur position en contact, cet effort étant exercé sur la surface de butée précitée.

Grâce aux procédés de l'invention, la vis est en permanence correctement positionnée par rapport aux composants constitutifs de l'élément prothétique, ce qui assure au chirurgien la précision de l'assemblage.

L'invention sera mieux comprise et d'autres avantages de celle-ci apparaîtront plus clairement à la lumière de la description qui va suivre de deux modes de réalisation d'un élément prothétique et de son procédé d'assemblage conformes à son principe, donnée uniquement à titre d'exemple et faite en référence aux dessins annexés dans lesquels :
- la figure 1 est une vue de côté éclatée d'un élément prothétique conforme à un premier mode de réalisation l'invention ;
- la figure 2 est une coupe axiale de l'élément prothétique de la figure 1, lors d'une première étape de son assemblage ;
- la figure 3 est une coupe analogue à la figure 2, lors d'une seconde étape d'assemblage ;
- la figure 4 est une coupe analogue à la figure 2, lors d'une troisième étape d'assemblage ;
- la figure 5 est une coupe analogue à la figure 2, au terme de l'assemblage ;
- la figure 6 est une vue analogue à la figure 3 pour un élément prothétique conforme à un second mode de réalisation de l'invention et
- la figure 7 est une vue analogue à la figure 5 pour l'élément de la figure 6.

L'élément prothétique E représenté sur les figures est destiné à être monté sur une épaule pour constituer une surface articulaire S destinée à coopérer avec une cupule non représentée appartenant à un élément prothétique complémentaire ancré dans l'humérus du patient.

La surface S est approximativement en forme de tronçon de sphère et constitue la surface externe d'un premier composant principal 1 réalisé dans un matériau compatible avec les mouvements d'articulation sur la cupule associée.

On note X₁ un axe central du composant 1 qui constitue un diamètre de la surface S. Le composant 1 est pourvu d'un évidemment central 11 centré sur l'axe X₁ et dont on note 12 la surface périphérique, cette surface étant de forme tronconique.

Un taraudage 13 est prévu au fond de l'évidemment 11, en étant centré sur l'axe X₁.

Un second taraudage 14 est centré sur l'axe X₁ et débouche vers l'extérieur, au niveau de la surface S.

Un alésage circulaire 15 est ménagé entre les taraudage 13 et 14, le rayon R₁₅ de cet alésage étant compris entre les rayons des taraudages 13 et 14.

Un second composant principal 2 est prévu pour être ancré dans la glène de l'épaule et comprend, pour ce faire, une tige 21 munie de stries 22. On note X₂ l'axe de la tige 21 qui est globalement cylindrique et à section circulaire.

Le composant 2 comprend également une platine 23, qui est pleine et monobloc avec la tige 21, cette platine étant pourvue d'une surface externe tronconique 24 centrée sur l'axe X₂.

Les rayons maximum des surfaces 12 et 24 sont sensiblement identiques et leurs angles de conicité respectifs, α et β, sont identiques, ce qui permet de prévoir un appui surfacique de ces surfaces l'une contre l'autre.

La tige 21 est pourvue d'un perçage central 25, de forme cylindrique et qui se prolonge dans la platine 23 par un taraudage 26.

L'élément E comprend également une vis 3 dont on note X₃ l'axe longitudinal. Cette vis est pourvue d'un premier filet 31 destiné à coopérer avec le taraudage 14 pour immobiliser la vis 3 sur le composant 1, les axes X₁ et X₃ étant alors confondus.

On note 32 l'extrémité de la vis 3 du côté du filet 31. Cette extrémité est pourvue d'un logement creux polygonal 33 pour la réception d'une clé de manoeuvre non représentée permettant de faire tourner la vis 3 autour de l'axe X₃.

La vis 3 est également pourvue d'une collerette radiale externe 34 dont on note R₃₄ le rayon.

Un second filet 35 est également prévu sur la vis 3, à l'opposé du filet 31 par rapport à la collerette 34. En d'autres termes, la collerette 34 est disposée axialement, le long de la vis 3, entre les filets 31 et 35.

On note respectivement R₃₁ et R₃₅ les rayons des filets 31 et 35. Le rayon R₃₄ est supérieur aux rayons R₃₁ et R₃₅, de telle sorte que la collerette 34 constitue un relief radial de la vis 3, notamment vis-à-vis des filets 31 et 35.

La vis 3 comporte, du côté de son extrémité arrondie 36 opposée à l'extrémité 32, une partie cylindrique 37 à section circulaire dont on note R₃₇ le rayon.

Les parties 36 et 37 de la vis 3 forment un doigt de guidage et de centrage destiné à coulisser dans le perçage 25 qui est à section circulaire et dont on note R₂₅ le rayon, ce rayon étant lui-même légèrement supérieur au rayon R₃₇. Les sections transversales respectives du logement 25 et de la partie 37 se correspondent l'une à l'autre, ce qui permet de guider en translation la partie 37, et par la même le doigt de centrage, lorsqu'il coulisse dans le logement 25.

Le taraudage 26 et le filet 35 sont prévus pour coopérer de façon à permettre un vissage de la vis 3 dans le composant 2 après introduction du doigt de centrage dans le perçage 25.

Le taraudage 26 est formé dans la platine 23, c'est-à-dire du côté d'introduction de la vis 3 dans le logement 25. La zone d'entrée 25a du logement 25 est de forme arrondie et concave, ce qui permet de pré-centrer l'extrémité 36 lors de son introduction dans le logement 25.

En variante, la zone 25a peut être remplacée par un chanfrein tronconique.

Une douille filetée 4 est également prévue et l'on note X₄ son axe central et 41 son filet extérieur qui est prévu pour coopérer avec le taraudage 13, afin d'immobiliser la douille 4 sur le composant 1, les axes X₁ et X₄ étant alors confondus.

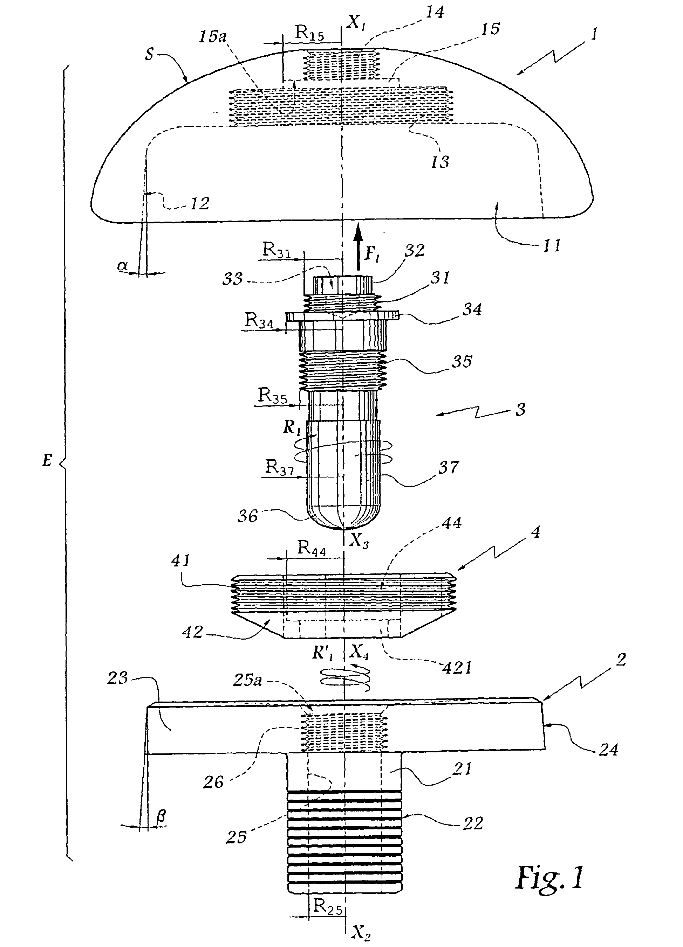

Cette douille comprend un anneau 42 dont on note 421 la surface radiale interne et 422 la surface latérale tournée vers la collerette 34 lorsque les pièces 3 et 4 sont dans la configuration de la figure 1. L'anneau 42 est monobloc avec le reste de la douille 4 et relié à celle-ci par des pattes 43 qui peuvent prendre toute forme adaptée à leur fonction.

La douille 4 est pourvue d'un alésage central 44 dont on note R₄₄ le rayon, ce rayon étant supérieur au rayon R₃₄.

L'assemblage de l'élément prothétique E a lieu de la façon suivante :
Tout d'abord, on introduit la vis 3 dans l'évidemment 11, dans le sens de la flèche F₁, jusqu'à faire prendre son filet 31 dans le taraudage 14. Il est alors possible de manoeuvrer la vis 3 en lui faisant subir un mouvement de rotation représenté par la flèche R₁, au moyen d'une clé mâle non représentée introduite dans le logement 33, par l'extérieur du composant 1.

Cette opération permet d'immobiliser la vis 3 sur le composant 1 en alignant les axes X₁ et X₃.

La collerette 34 est alors reçue dans l'alésage 15 et porte contre le fond 15a de celui-ci. Pour ce faire, le rayon R₁₅ est légèrement supérieur au rayon R₃₄.

Il est alors possible de visser la douille 4 dans le taraudage 13, comme représenté par la flèche R'₁ l'anneau 42 étant alors disposé autour de la vis 3.

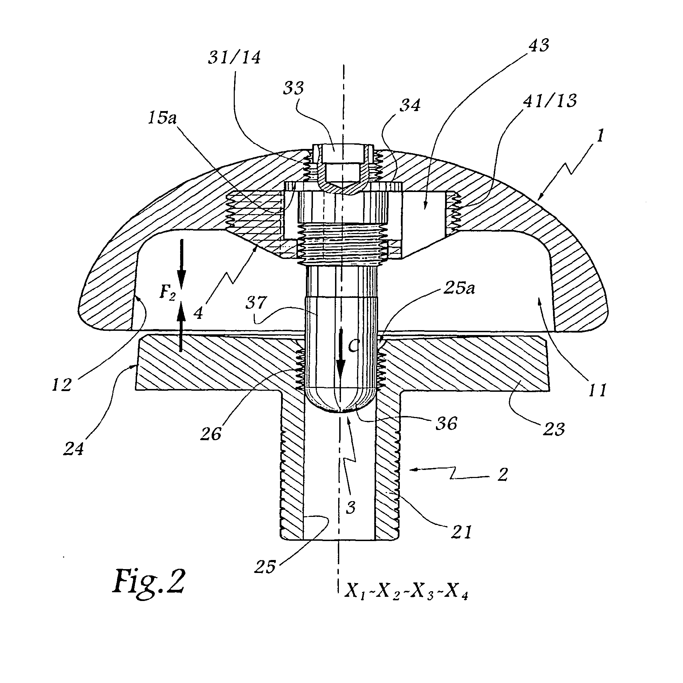

On peut alors rapprocher les composants 1 et 2 comme représentés par les flèches F₂ à la figure 2, ce qui revient à faire pénétrer et coulisser le doigt de guidage et de centrage 36-37 dans le perçage 25, comme représenté par la flèche de coulissement C. Ceci induit un alignement de l'axe X₂ sur les axes X₁ et X₃.

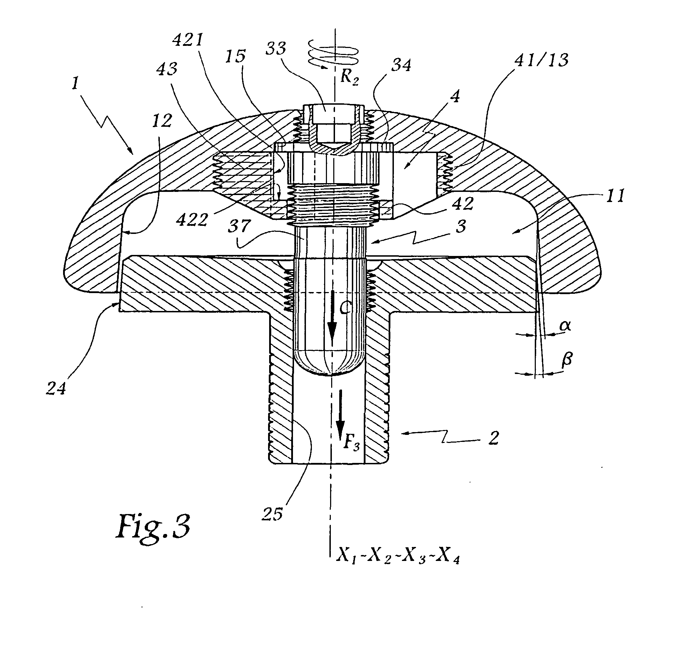

On peut alors faire progresser les composants 1 et 2 dans le sens des flèches F₂ jusqu'à amener en contact les surfaces 12 et 24, comme représenté à la figure 3.

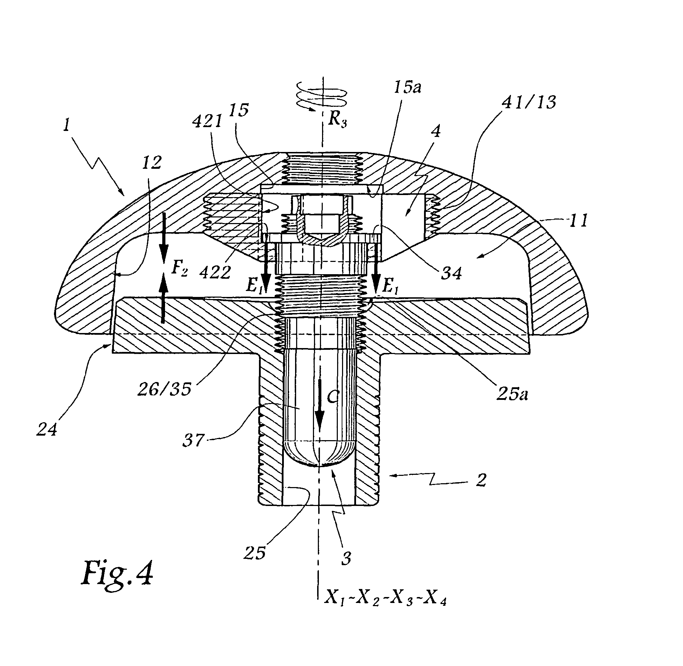

Il est alors possible de dévisser la vis 3 par rapport au taraudage 14, comme représenté par la flèche R₂ à la figure 3, puis de pousser la vis 3 en direction du composant 2 comme représenté par la flèche F₃, ce qui a pour effet d'amener le filet 35 au contact du taraudage 26, la collerette 34 étant alors en appui contre la surface 422 de l'anneau 42, comme représenté à la figure 4.

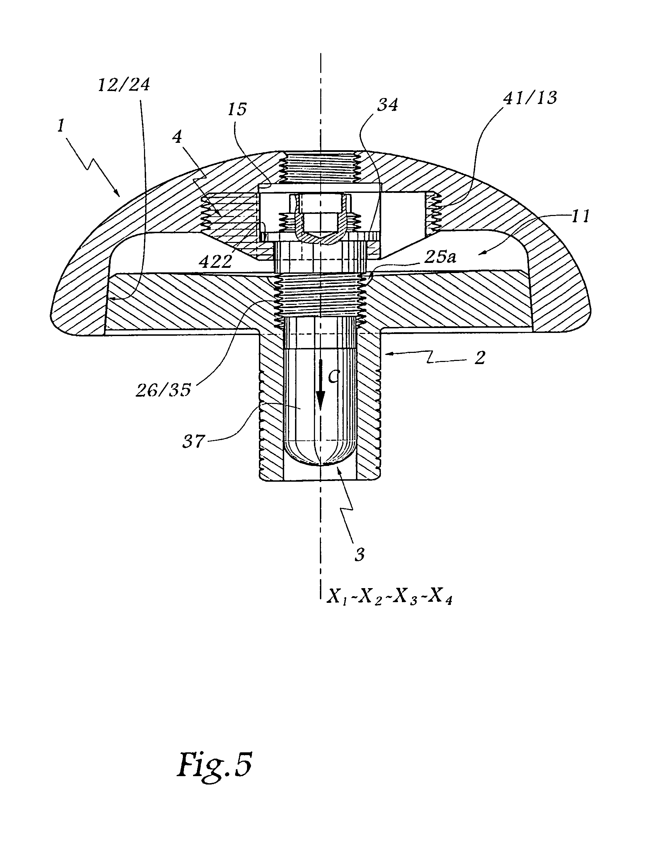

Il est alors possible de visser la vis 3 dans le composant 2 grâce au filet 35 et au taraudage 26, comme représenté par la flèche R₃ à la figure 4, ce qui a pour effet de rapprocher encore les composants 1 et 2, comme représenté par les flèches F₂. Ceci permet de passer de la configuration de la figure 4 à celle de la figure 5, où les surfaces 12 et 24 sont en appui ferme l'une contre l'autre, ce qui garantit l'immobilisation relative des composants 1 et 2.

Les filets 35 et 41 sont de sens opposés, ce qui évite un dévissage intempestif de la douille 4 lors du vissage de la vis 3 dans le taraudage 26.

Au cours du vissage de la vis 3 sur le composant 2, la collerette 34 exerce sur la surface 422 de l'anneau 42 un effort E₁ parallèle aux axes X₁, X₂ et X₃ et dirigé vers le composant 2, cet effort étant transmis par la douille 4 au composant 1 qui progresse ainsi en direction de la platine 23.

En variante, dans la position de contact entre le filet 35 et le taraudage 26, la collerette 34 peut être au voisinage de la surface 422. Dans ce cas, le début du vissage de la vis 3 sur le composant 2 induit un rapprochement de la collerette 34 et de l'anneau 42, avant que l'effort E₁ ne soit appliqué.

Au cours des phases d'assemblage de l'élément E représenté respectivement aux figures 2 à 5, et comme représenté par la flèche C, le doigt de guidage constitué, pour l'essentiel, de la partie cylindrique 37 de la vis 3 coulisse dans le perçage 25 en guidant efficacement la vis 3 par rapport au composant 2, ce qui évite les défauts d'alignement des axes X₁, X₂ et X₃ et garantit ainsi un bon positionnement relatif des surfaces de portée tronconiques 12 et 24.

Selon une autre approche, l'assemblage de l'élément E peut être obtenu, à partir de la configuration de la figure 2 en impactant le composant principal 1 en direction du composant principal 2 de telle sorte que la partie cylindrique 37 de la vis 3, est pour l'essentiel, enfoncée dans le logement 25. L'impaction a lieu jusqu'à ce que les composants 1 et 2 viennent en contact. Il est alors possible de dévisser la vis 3 par rapport au composant 1 et de la visser dans le taraudage 26, ce qui permet d'exercer, sur la surface 422 et grâce à la collerette 34, un effort d'immobilisation relative des composants 1 et 2.

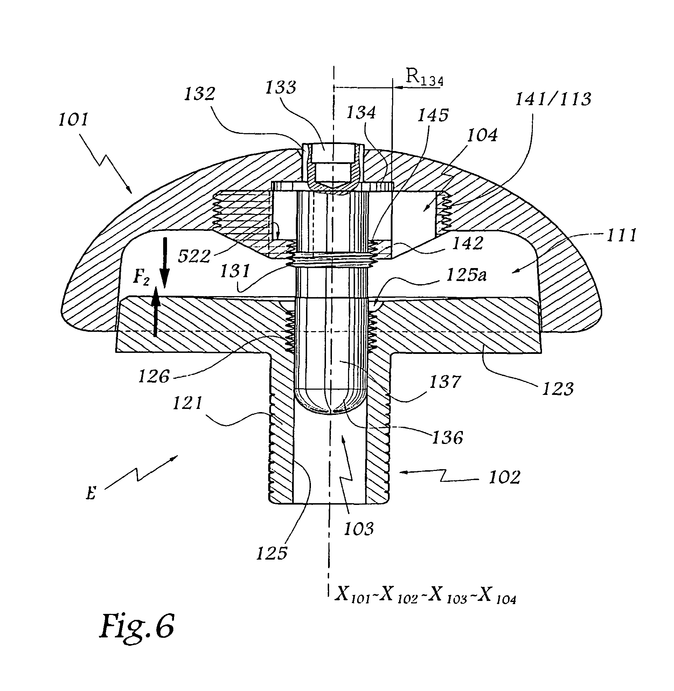

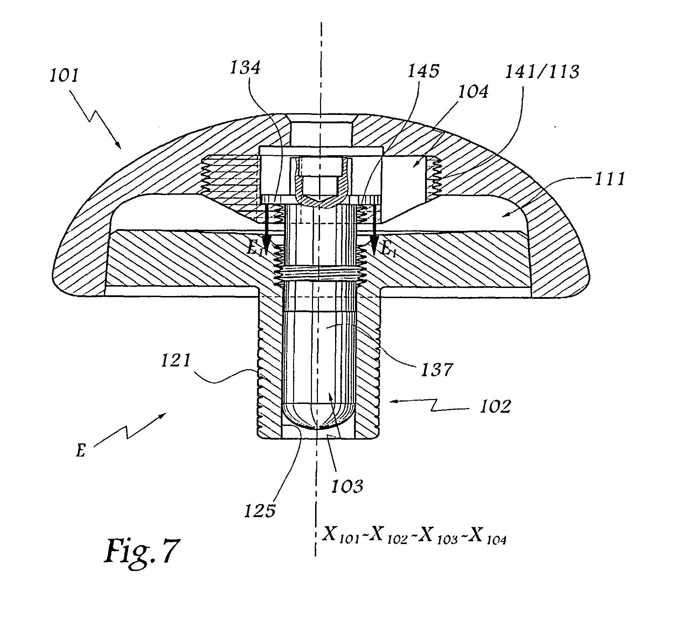

Dans le second mode de réalisation de l'invention représenté aux figures 6 et 7, les éléments analogues à ceux du premier mode de réalisation portent des références identiques augmentées de 100. L'élément prothétique E de ce mode de réalisation comprend deux composants principaux 101 et 102 ainsi qu'une vis 103 et une douille 104. Le composant 102 est prévu pour être engagé dans un évidemment central 111 du composant 101. On note respectivement X₁₀₁, X₁₀₂, X₁₀₃ et X₁₀₄ les axes centraux des éléments 101 à 104. Ces axes sont confondus dans les positions des figures 6 et 7. La douille 104 est pourvue d'un filet 141 permettant son vissage dans un taraudage 113 prévu dans le composant 101. La douille 104 est également pourvue d'un taraudage central 145 prévu pour coopérer avec un filet 131 ménagé sur la vis 103.

Comme précédemment, la vis 103 est munie, au niveau de son extrémité supérieure 132, d'un logement 133 d'une clé de manoeuvre. Une collerette 134 de rayon R₁₃₄ relativement important est également prévue sur la vis 103, cette collerette pouvant venir en appui contre une surface latérale 522 d'un anneau 142 formé par la douille 104.

Un perçage 125 est ménagé dans la tige d'ancrage 121 du composant 102 et traverse une platine 123 destinée à être engagée dans un évidemment central 111 du composant 101.

Le perçage 125 est pourvu, à proximité de sa zone d'entrée 125a d'un taraudage 126 orienté et configuré pour pouvoir coopérer avec le filet 131 de la vis 103.

Une partie cylindrique 137 et une extrémité 136 de la vis 103 constituent un doigt de guidage et de centrage de cette vis dans le logement 125.

Le fonctionnement est le suivant :
Pour l'assemblage de l'élément E, on introduit la vis 103 dans la douille 104 en faisant traverser à l'extrémité 136 un évidemment central de la douille 104, cet évidemment étant bordé par le taraudage 145. On visse alors la vis 103 dans le taraudage 145 et le sous-ensemble 103-104 ainsi réalisé est vissé sur le composant 101 grâce au filet 141 et au taraudage 113. Il est alors possible de précentrer, par la coopération du doigt 136-137 et du logement 125, le composant 101 par rapport au composant 102, éventuellement déjà en place sur un patient, puis de rapprocher ces éléments comme représenté par les flèches F₂ à la figure 6.

Il est alors possible de dévisser la vis 103 par rapport à la douille 104 tout en la poussant en direction de la platine 123, la translation de la vis 103 par rapport au composant 102 étant guidée par le doigt 136-137. On peut alors rapprocher les composants 101 et 102, comme représenté à la figure 6, et visser la vis 103 dans le taraudage 126 grâce au filet 131, ce qui permet d'obtenir une immobilisation ferme des éléments 102 et 103 l'un par rapport à l'autre, grâce à un effort E₁ exercé par la collerette 134 sur la surface 522.

Comme mentionné en référence au premier mode de réalisation, un autre procédé d'assemblage peut consister, à partir de la position de la figure 6 à impacter le composant 101 en direction du composant 102 de façon à amener en contact ces composants. La vis 103 est alors dévissée par rapport à la douille 4 puis vissée dans le taraudage 126 pour maintenir ces composants en contact l'un avec l'autre, grâce à l'effort E₁.

Le filet 131 du second mode de réalisation remplit les fonctions des filets 31 et 35 du premier mode de réalisation.

L'invention a été représentée avec un composant prothétique destiné à équiper la glène d'une épaule. Elle est cependant applicable à tout type d'élément prothétique comprenant deux composants principaux.

## Revendications

1. Elément prothétique comprenant un premier et un second composants principaux (1, 2 ; 101, 102) destinés à être assemblés ensemble et immobilisés l'un par rapport à l'autre au moyen d'une vis (3 ; 103), ladite vis comportant au moins un relief radial (34 ; 134), apte à venir en appui (E₁) contre une surface de butée (422 ; 522) solidaire dudit premier composant (1 ; 101), et une extrémité non filetée (36-37 ; 136-137) formant doigt de guidage et apte à coulisser (C) dans un logement (25 ; 125) ménagé dans ledit second composant (2 ; 102), **caractérisé en ce que** ladite vis est apte à être vissée au moyen d'au moins un filet (31, 35 ; 131) dans deux taraudages (14, 26 ; 126, 145) solidaires ou destinés à être rendus solidaires respectivement de l'un (1 ; 101) et de l'autre (2 ; 102) composants principaux.

2. Elément prothétique selon la revendication 1, **caractérisé en ce que** ledit relief est formé par une collerette radiale (34) ménagée sur ladite vis (3), entre lesdits deux filets (31, 35).

3. Elément prothétique selon l'une des revendications précédentes, **caractérisé en ce que** ladite surface de butée (422 ; 522) est annulaire et entoure ladite vis (3 ; 103).

4. Elément prothétique selon la revendication 3, **caractérisé en ce que** ladite surface de butée est formée par une face (422 ; 522) d'un anneau (42 ; 142) entourant ladite vis (3 ; 103), ledit anneau étant rigidement lié à une douille (4 ; 104) vissée dans ledit premier composant (1 ; 101).

5. Elément selon l'une des revendications précédentes, **caractérisé en ce que** lesdits premier et second composants principaux (1, 2 ; 101, 102) sont respectivement pourvus de surfaces de portée tronconiques (12, 24), ladite vis (3 ; 103) étant apte à plaquer, par son vissage (R₃), lesdites surfaces l'une contre l'autre.

6. Elément prothétique selon la revendication 5, **caractérisé en ce que** lesdites surfaces de portée tronconiques (12, 24) sont respectivement centrées sur un axe central de ladite vis (X₁, X₃), lorsqu'elle est vissée dans ledit premier composant (1 ; 101), et sur un axe central (X₂) dudit logement de coulissement (C) dudit doigt de centrage (36-37 ; 136-137).

7. Elément prothétique selon l'une des revendications précédentes, **caractérisé en ce que** le taraudage (26 ; 125) solidaire ou destiné à être rendu solidaire dudit second composant (2 ;: 102) est formé dans ledit logement (25 ; 125), du côté d'une zone d'entrée (25a ; 125a) de ladite vis (3 ; 103).

8. Elément prothétique selon l'une des revendications précédentes, **caractérisé en ce que** ledit premier composant (1 ; 101) forme une surface articulaire convexe (S) et définit un volume (11 ; 111) dans lequel peut être montée ladite vis (3 ; 103) et engagé, au moins partiellement, ledit second composant (2 : 102).

9. Elément prothétique selon l'une des revendications précédentes, **caractérisé en ce que** ledit logement (25 ; 125) de coulissement (C) dudit doigt de guidage (36-37 ; 136-137) est ménagé dans une tige (21 ; 121) d'ancrage dudit second composant (2 ; 102) dans un os.

10. Elément prothétique selon l'une des revendications précédentes, **caractérisé en ce que** ladite vis (3) est pourvue de deux filets aptes à coopérer successivement avec deux taraudages (14, 26) respectivement solidaires de l'un (1) et de l'autre (2) composants principaux.

11. Elément prothétique selon l'une des revendications 1 à 9, **caractérisé en ce que** ladite vis (103) est pourvue d'un unique filet (131) apte à coopérer successivement avec deux taraudages (126 ; 145) solidaires ou destinés à être rendus solidaires respectivement de l'un (101) et de l'autre (102) composants principaux.

12. Utilisation d'un élément prothétique (E) selon l'une des revendications précédentes, pour la réalisation de la partie glénoïdienne d'une prothèse totale d'épaule.

## Claims

1. Prosthetic element comprising a first and a second main components (1, 2; 101, 102) which are designed to be assembled together and immobilised relative to one another by means of a screw (3; 103), the said screw comprising at least one radial relief (34; 134) which can be supported (E₁) against a stop surface (422; 522) which is integral with the said first component (1; 101), and a non-threaded end (36-37; 136-137) which forms a guide finger, and can slide (C) in a receptacle (25; 125) provided in the said second component (2; 102), **characterised in that** the said screw can be screwed by means of at least one thread (31, 35; 131) into two female threads (14, 26; 126, 145) which are integral, or are designed to be rendered integral, respectively with one (1; 101) and the other (2; 102) of the main components.

2. Prosthetic element according to claim 1, **characterised in that** the said relief is formed by a radial flange (34) provided on the said screw (3) between the said two threads (31, 35).

3. Prosthetic element according to either of the preceding claims, **characterised in that that** said stop surface (422; 522) is annular and surrounds the said screw (3; 103).

4. Prosthetic element according to claim 3, **characterised in that** the said stop surface is formed by a face (422; 522) of a ring (42; 142) which surrounds the said screw (3; 103), the said ring being rigidly connected to a bush (4; 104) which is screwed into the said first component (1; 101).

5. Element according to any one of the preceding claims, **characterised in that** the said first and second main components (1, 2; 101, 102) are provided respectively with frusto-conical support surfaces (12, 24), the said screw (3; 103) being able to lay the said surfaces against one another by means of its screwing (R₃).

6. Prosthetic element according to claim 5, **characterised in that** the said frusto-conical support surfaces (12, 24) are centred respectively on a central axis of the said screw (X₁, X₂) when it is screwed into the said first component (1; 101), and on a central axis (X₂) of the said sliding receptacle (C) of the said centring finger (36-37; 136-137).

7. Prosthetic element according to any one of the preceding claims, **characterised in that** the female thread (26; 126) which is integral or is designed to be rendered integral with the said second component (2; 102) is formed in the said receptacle (25; 125), on the side of an intake area (25a; 125a) of the said screw (3; 103).

8. Prosthetic element according to any one of the preceding claims, **characterised in that** the said first component (1; 101) forms a convex articular surface (5) and defines a volume (11; 111) in which the said screw (3; 103) can be fitted and the said second component (2; 102) can be engaged at least partially.

9. Prosthetic element according to any one of the preceding claims, **characterised in that** the said receptacle (25; 125) for sliding (C) of the said guide finger (36-37; 136-137) is provided in a rod (21; 121) for anchorage of the said second component (2; 102) in a bone.

10. Prosthetic element according to any one of the preceding claims, **characterised in that** the said screw (3) is provided with two threads which can co-operate successively with two female threads (14, 26) which are integral respectively with one (1) and the other (2) of the main components.

11. Prosthetic element according to any one of claims 1 to 9, **characterised in that** the said screw (103) is provided with a single thread (131) which can co-operate successively with two female threads (126; 145) which are integral, or are designed to be rendered integral, respectively with one (101) and the other (102) of the main components.

12. Use of a prosthetic element (E) according to any one of the preceding claims, for production of the glenoid part of a full shoulder prosthesis.

## Patentansprüche

1. Prothesenelement, das einen ersten und einen zweiten Hauptbestandteil (1, 2; 101, 102) aufweist, die dazu bestimmt sind, gemeinsam zusammengebaut und zueinander mittels einer Schraube (3; 103) festgelegt zu werden, wobei die Schraube mindestens ein Radialrelief (34; 134) aufweist, das gegen eine Anschlagfläche (422; 522), die fest mit dem ersten Bestandteil (1; 101) verbunden ist, zum Aufliegen (E₁) kommen kann, und ein Ende (36-37; 136-137) ohne Gewinde, das einen Führungsfinger bildet und in einer Aufnahme (25; 125), die in dem zweiten Bestandteil (2; 102) eingerichtet ist, gleiten (C) kann, **dadurch gekennzeichnet, dass** die Schraube mittels zumindest eines Gewindes (31, 35; 131) in zwei Innengewinde (14, 26; 126, 145), die jeweils mit dem einen (1; 101) und dem anderen (2; 102) Hauptbestandteil fest verbunden sind oder dazu bestimmt sind, damit fest verbunden zu werden, geschraubt werden kann.

2. Prothesenelement nach Anspruch 1, **dadurch gekennzeichnet, dass** das Relief aus einem radialen Kragen (34) gebildet ist, der auf der Schraube (3) zwischen den zwei Gewinden (31, 35) eingerichtet ist.

3. Prothesenelement nach einem der vorhergehenden Ansprüche, **dadurch gekennzeichnet, dass** die Anschlagfläche (422; 522) ringförmig ist und die Schraube (3; 103) umgibt.

4. Prothesenelement nach Anspruch 3, **dadurch gekennzeichnet, dass** die Anschlagfläche aus einer Fläche (422; 522) eines Rings (42; 142) gebildet ist, der die Schraube (3; 103) umgibt, wobei der Ring steif mit einer Hülse (4; 104) verbunden ist, die in den ersten Bestandteil (1; 101) geschraubt ist.

5. Prothesenelement nach einem der vorhergehenden Ansprüche, **dadurch gekennzeichnet, dass** der erste und der zweite Hauptbestandteil (1, 2; 101, 102) jeweils mit kegelstumpfförmigen Auflageflächen (12, 24) versehen sind, wobei die Schraube (3; 103) durch ihr Schrauben (R₃) die Flächen gegeneinander drücken kann.

6. Prothesenelement nach Anspruch 5, **dadurch gekennzeichnet, dass** die kegelstumpfförmigen Auflageflächen (12, 24) jeweils auf eine zentrale Achse (X₁, X₃) der Schraube zentriert sind, wenn sie in den ersten Bestandteil (1; 101) geschraubt ist, und auf eine zentrale Achse (X₂) der Gleitaufnahme (C) des Zentrierfingers (36-37; 136-137).

7. Prothesenelement nach einem der vorhergehenden Ansprüche, **dadurch gekennzeichnet, dass** das Innengewinde (26; 126), das fest mit dem zweiten Bestandteil (2; 102) verbunden ist oder dazu bestimmt ist, fest mit dem zweiten Bestandteil (2; 102) verbunden zu werden, in der Aufnahme (25; 125) an der Seite einer Eingangszone (25a; 125a) für die Schraube (3; 103) ausgebildet ist.

8. Prothesenelement nach einem der vorhergehenden Ansprüche, **dadurch gekennzeichnet, dass** der erste Bestandteil (1; 101) eine konvexe Anlenkfläche (S) bildet und ein Volumen (11; 111) definiert, in das die Schraube (3; 103) montiert und zumindest zum Teil der zweite Bestandteil (2; 102) eingreifen kann.

9. Prothesenelement nach einem der vorhergehenden Ansprüche, **dadurch gekennzeichnet, dass** die Aufnahme (25; 125) zum Gleiten (C) des Führungsfingers (36-37; 136-137) in einem Schaft (21; 121) zum Verankern des zweiten Bestandteils (2; 102) in einem Knochen eingerichtet ist.

10. Prothesenelement nach einem der vorhergehenden Ansprüche, **dadurch gekennzeichnet, dass** die Schraube (3) mit zwei Gewinden versehen ist, die nacheinander mit zwei Innengewinden (14, 26), die jeweils fest mit dem einen (1) und dem anderen (2) Hauptbestandteil verbunden sind, zusammenwirken können.

11. Prothesenelement nach einem der Ansprüche 1 bis 9, **dadurch gekennzeichnet, dass** die Schraube (103) mit einem einzigen Gewinde (131) versehen ist, das nacheinander mit zwei Innengewinden (126; 145), die jeweils mit dem einen (101) und dem anderen (102) Hauptbestandteil fest verbunden sind oder dazu bestimmt sind, damit fest verbunden zu werden, zusammenwirken kann.

12. Einsatz eines Prothesenelements (E) nach einem der vorhergehenden Ansprüche zum Herstellen des glenoidalen Teils einer Totalschulterprothese.
